(19) Europäisches Patentamt
European Patent Office
Office européen des brevets

(11) Veröffentlichungsnummer: **0 450 359 B1**

(12) **EUROPÄISCHE PATENTSCHRIFT**

(45) Veröffentlichungstag der Patentschrift: **15.03.95**

(51) Int. Cl.[6]: **A01N 43/58**, A01N 47/06, A01N 47/18, //C07D237/12, C07D237/14

(21) Anmeldenummer: **91103902.2**

(22) Anmeldetag: **14.03.91**

# (54) Herbizides Mittel.

(30) Priorität: **05.04.90 AT 807/90**

(43) Veröffentlichungstag der Anmeldung:
**09.10.91 Patentblatt 91/41**

(45) Bekanntmachung des Hinweises auf die Patenterteilung:
**15.03.95 Patentblatt 95/11**

(84) Benannte Vertragsstaaten:
**AT BE CH DE DK ES FR GB GR IT LI LU NL SE**

(56) Entgegenhaltungen:
**FR-A- 2 141 253**
**FR-A- 2 207 133**
**FR-A- 2 234 296**
**GB-A- 2 047 702**
**JP-A-56 113 767**

**DERWENT CENTRAL PATENTS INDEX, BASIC ABSTRACTS JOURNAL, SCHNITT C:AGDOC, Woche 8407, 11. April 1984, Derwent Publications Ltd, London, GB; & JP-A-59 001 469 (NIPPON SODA K.K.) 06-01-1984**

(73) Patentinhaber: **SANDOZ LTD.**
**Lichtstrasse 35**
**CH-4002 Basel (CH)**
(84) Benannte Vertragsstaaten:
**BE CH DK ES FR GB GR IT LI LU NL SE**

(73) Patentinhaber: **SANDOZ-PATENT-GMBH**
**Humboldtstrasse 3**
**D-79539 Lörrach (DE)**
(84) Benannte Vertragsstaaten:
**DE**

(73) Patentinhaber: **SANDOZ-ERFINDUNGEN Verwaltungsgesellschaft m.b.H.**
**Brunner Strasse 59**
**A-1230 Wien (AT)**
(84) Benannte Vertragsstaaten:
**AT**

(72) Erfinder: **Kores, Dietmar, Dr.**
**Gartenlehnerstrasse 11**
**A-4060 Leonding (AT)**
Erfinder: **Tramberger, Hermann**
**Dorf 28**
**A-3353 Seitenstetten (AT)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

Erfinder: **Wörther, Rudolf Helmut, Dr.**
**Spaunstrasse 82**
**A-4020 Linz (AT)**
Erfinder: **Jellinger, Rudolf**
**Im Neugereith 12**
**A-4030 Linz (AT)**
Erfinder: **Kloimstein, Engelbert, Ing.**
**Schiferplatz 22**
**A-4070 Eferding (AT)**
Erfinder: **Schönbeck, Rupert, Dr.**
**Ortmayrstrasse 26**
**A-4060 Leonding (AT)**

## Beschreibung

Die Erfindung betrifft ein Verfahren zur Bekämpfung von Unkräutern mit Hilfe bestimmter substituierter Phenylpyridazinderivate und deren Verwendung als Wirkstoff in einem herbiziden Mittel.

Aus JP-A 84/212 479 sind herbizid wirksame 3-Arylpyridazine bekannt, die in 5-Stellung am Pyridazinring eine Alkyl- oder Dialkyl- oder Alkylalkoxy-oder Dialkylalkoxyaminogruppe aufweisen.

Ferner sind aus JP-A 81/113767 substituierte Aryl-4(O-Acyl)-6-halogenpyridazine mit fungizider Wirksamkeit bekannt.

Es konnte nun überraschenderweise gefunden werden, daß solche in JP-A 81/113767 beschriebenen substituierten Phenylpyridazinderivate eine ausgezeichnete herbizide Wirksamkeit aufweisen.

Gegenstand der Erfindung ist daher ein Verfahren zur Bekämpfung von Unkräutern, das dadurch gekennzeichnet ist, daß man mindestens eine Verbindung der Formel

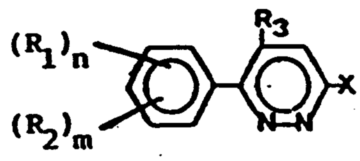

I

in der $R_1$ und $R_2$ unabhängig voneinander jeweils die Reste Hydroxy, Halogen, Cyano, Nitro, Amino das gegebenenfalls durch Alkyl mit 1 - 4 C-Atomen substituiert sein kann, geradkettiges oder verzweigtes Alkyl mit 1 - 10 C-Atomen, das gegebenenfalls durch Hydroxy, Halogen oder Cyano ein- oder mehrfach substituiert sein kann, geradkettiges oder verzweigtes Alkoxy mit 1 - 10 C-Atomen, Cycloalkyl mit 5 - 7 C-Atomen, Cycloalkoxy mit 5 - 7 C-Atomen, Phenyl, Phenoxy, Alkylthio oder Alkylsulfonyl mit 1 - 4 C-Atomen bedeuten,
n und m unabhängig voneinander jeweils die Zahlen 0, 1, 2 oder 3 bedeuten, wobei n + m die Zahlen 1 bis 5 bedeuten $R_3$ die Reste Hydroxy, Halogen, $-OC(O)ZR_4$ oder $-OC(O)NR_4R_5$ bedeutet, wobei
$R_4$ einen geradkettigen oder verzweigten, gesättigten oder ungesättigten Alkylrest mit 1 - 15 C-Atomen, der gegebenenfalls durch einen Phenylrest substituiert sein kann oder einen Phenylrest oder einen Cycloalkylrest mit 5 - 7 C-Atomen,
$R_5$ einen geradkettigen oder verzweigten, gesättigten oder ungesättigten Alkylrest mit 1 - 15 C-Atomen, der gegebenenfalls durch einen Phenylrest substituiert sein kann oder einen Phenylrest oder einen Cycloalkylrest mit 5 - 7 C-Atomen,
Z eine Einfachbindung, Sauerstoff oder Schwefel
und X Halogen bedeutet oder deren pflanzenphysiologisch verträgliche Salze auf Unkräuter oder deren Lebensraum einwirken läßt.

In der Formel I bedeuten $R_1$ und $R_2$ unabhängig voneinander Hydroxy, eine Cyanogruppe, eine Nitrogruppe, eine Aminogruppe, die gegebenenfalls durch Alkyl mit 1 - 4 C-Atomen substituiert sein kann, wie einen Methylamino-, Ethylamino-, Propylamino-, Butylamino-, einen Dimethylamino-, Diethylamino-, Methyl-ethylaminorest und dgl. oder ein Halogenatom, beispielsweise ein Chlor-, Brom- oder Fluoratom. Weiters können $R_1$ und $R_2$ unabhängig voneinander einen geradkettigen oder verzweigten Alkylrest mit 1 - 10 C-Atomen, beispielsweise einen Methyl-, Ethyl-, Propyl-, Butyl-, Pentyl-, Hexyl-, Heptyl-, Octyl-, Nonyl- oder Decylrest oder deren verzweigte Analoge, wie einen i-Propyl-, i-Butyl-, t-Butylrest oder dgl. bedeuten. Diese Alkylreste können ein- oder mehrfach durch Hydroxy, Cyano- oder Halogen, beispielsweise Chlor, Brom oder Fluor substituiert sein. Beispiele für solche Reste sind Cyanoalkylreste, wie der Cyanomethylrest, Hydroxalkylreste, wie der Hydroxyethylrest oder Halogenalkylreste, wie der Chlormethylrest, der Brommethylrest oder der Trifluormethylrest. Weiters können $R_1$ und $R_2$ unabhängig voneinander einen Alkoxyrest mit 1 - 10 C-Atomen in der Alkylkette, beispielsweise einen Methoxy-, einen Ethoxy-, einen Propoxy-, einen Butoxyrest und dgl., oder einen Cycloalkylrest mit 5 - 7 C-Atomen, wie einen Cyclopentyl-, Cyclohexyl- oder Cycloheptylrest, oder einen Phenylrest oder einen Phenoxyrest bedeuten. $R_1$ und $R_2$ können ferner jeweils einen Alkylthiorest mit 1 - 4 C-Atomen in der Alkylkette, wie einen Methylthio-, Ethylthio-, Propylthio- oder Butylthiorest, oder einen Alkylsulfonylrest mit 1 - 4 C-Atomen, beispielsweise einen Methylsulfonylrest, bedeuten.

n und m bedeuten jeweils die Zahlen 0, 1, 2 oder 3, wobei n + m die Zahlen 1 bis 5 bedeutet.

Bevorzugt sind jene Verbindungen, in denen $R_1$ und $R_2$ jeweils ein Halogenatom, beispielsweise Chlor, Brom oder Fluor und n + m die Zahlen 1 oder 2 bedeuten oder einen Alkylrest mit 1 - 4 C-Atomen, der gegebenenfalls ein- oder mehrfach durch Halogen, vorzugsweise durch Chlor oder Fluor substituiert ist und

n + m die Zahlen 1 oder 2 bedeuten, oder einen Alkoxyrest mit 1 - 4 C-Atomen in der Alkylkette oder einen Cyclohexylrest bedeuten.

Besonders bevorzugt sind jene Verbindungen, in denen $R_1$ und $R_2$ ein Halogenatom, beispielsweise Chlor oder Fluor, wobei n + m 1 oder 2 ist, oder einen Trifluormethylrest, wobei n + m die Zahl 1 ist, bedeuten.

$R_3$ bedeutet Hydroxy oder den Rest -OC(O)Z$R_4$, oder den Rest -OC(O)N$R_4R_5$, wobei $R_4$ einen geradkettigen oder verzweigten gesättigten oder ungesättigten Alkylrest mit 1 - 15 C-Atomen, beispielsweise einen Methyl-, Ethyl-, Propyl- oder Butyl-, Pentyl-, Heptyl-, Decyl-, Dodecyl-, Pentadecylrest, oder deren verzweigte Analoga bedeutet, der gegebenenfalls durch einen Phenylrest substituiert sein kann. Ferner kann $R_4$ einen Phenylrest oder einen Cycloalkylrest mit 5 - 7 C-Atomen, beispielsweise den Cyclohexylrest bedeuten. $R_5$ bedeutet einen geradkettigen oder verzweigten, gesättigten oder ungesättigten Alkylrest mit 1 - 15 C-Atomen, beispielsweise einen Methyl-, Ethyl-, Propyl- oder Butyl-, Pentyl-, Heptyl-, Decyl-, Dodecyl-, Pentadecylrest, oder deren verzweigte Analoga, der gegebenenfalls durch einen Phenylrest substituiert sein kann. Ferner kann R5 einen Phenylrest oder eine Cycloalkylrest mit 5 - 7 C-Atomen beispielsweise den Cyclohexylrest bedeuten. Die Reste $R_4$ und $R_5$ können gleich oder verschieden sein. Bevorzugt sind jene Verbindungen, in denen $R_3$ den Rest -OC(O)Z$R_4$ bedeutet, wobei $R_4$ und Z die oben angegebene Bedeutung haben.

X bedeutet Halogen, vorzugsweise Chlor oder Brom.

Die Verbindungen der Formel I können hergestellt werden, in dem man

a) ein entsprechend substituiertes 3-Phenyl-pyridazinon-6 der Formel II

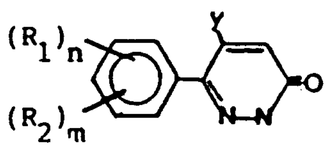

II

in der $R_1$, $R_2$, n und m die oben angegebene Bedeutung haben und Y Wasserstoff oder Chlor bedeutet mit Phosphoroxidhalogenid und, falls Y Wasserstoff bedeutet, zusätzlich mit Phosphor oder $PCl_3$ oder $PCl_5$ und einem Halogenierungsmittel zur entsprechenden Dihalogenverbindung der Formel III

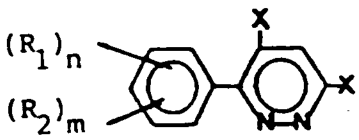

III

in der X die oben angegebene Bedeutung hat, umsetzt,

b) diese gegebenenfalls in Gegenwart eines Verdünnungsmittels und in Gegenwart einer Base zum entsprechenden 3-Phenyl-4-hydroxy-6-halogenpyridazin der Formel IV umsetzt,

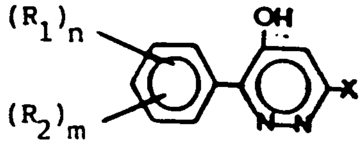

IV

in der X die oben angegebene Bedeutung hat, worauf man

c) gegebenenfalls diese Verbindung der Formel IV in Gegenwart eines organischen Verdünnungsmittels und in Gegenwart einer Base, beispielsweise Natriumhydroxid, Kaliumhydroxid, oder Triethylamin mit einem entsprechenden Säurehalogenid der Formel V oder Va

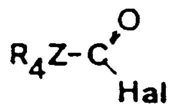 V

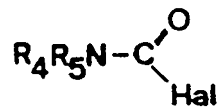 Va

in der $R_4$, $R_5$ und Z die oben angegebene Bedeutung haben,
zu den Verbindungen der Formel I, in der $R_3$ nicht Hydroxy und nicht Halogen bedeutet, umsetzt.

Man kann allerdings auch in Schritt a) von der entsprechenden 3-Aryl-6-halogenpyridazinverbindung ausgehen.

Bedeutet $R_1$ oder $R_2$ eine Nitrogruppe, kann man diese gegebenenfalls auch vor Durchführung des Reaktionsschrittes c) durch Nitrieren der entsprechenden 3-Aryl-4-hydroxy-6-halogenpyridazinverbindung einführen. Es ist aber auch möglich, bereits in Schritt a) das entsprechend substituierte Pyridazinon-6 einzusetzen.

Die als Ausgangsverbindungen benötigten substituierten 3-Phenylpyridazinone-6 werden in an sich bekannter Weise aus dem entsprechenden Acetophenon durch Umsetzung mit Glyoxylsäure und Hydrazin oder aus der entsprechenden Benzoylacrylsäure durch Umsetzung mit einem niederen aliphatischen Alkohol in Gegenwart eines Katalysators und anschließender Umsetzung mit Hydrazin oder aus der entsprechenden Benzoylacrylsäure durch Veresterung mit einem niederen aliphatischen Alkohol in Gegenwart eines Veresterungskatalysators und anschließender Umsetzung mit Hydrazin bzw. durch Umsetzung der entsprechenden Benzoylacrylsäuren mit $NH_3$ und Hydrazin erhalten.

Die Herstellung der Verbindungen der Formel II, in der Y Cl bedeutet, kann gemäß Weissberger A., Taylor E. C. (Hersg.), The Chemistry of Heterocyclic Compounds Vol. 28, p. 32 erfolgen. Die Umsetzung zu den Dihalogenverbindungen erfolgt in Gegenwart von Phosphoroxidhalogenid und falls Y H bedeutet, zusätzlich durch Umsetzen mit Phosphor oder $PCl_3$ oder $PCl_5$ und einem Halogenierungsmittel. Die Dihalogenverbindungen werden anschließend gegebenenfalls in Gegenwart eines Verdünnungsmittels, beispielsweise Dioxan/Wassergemisch Tetrahydrofuran u. dgl. und in Gegenwart einer Base beispielsweise einem Alkalihydroxid wie Natronlauge, Kalilauge zu den Verbindungen der Formel IV umgesetzt.

Der gegebenenfalls anschließende Acylierungsschritt durch Umsetzung mit einem Säurehalogenid der Formel V oder Va, erfolgt in Gegenwart eines organischen Verdünnungsmittels, beispielsweise in wäßrigem Aceton, Toluol oder Benzol u. dgl. und in Gegenwart einer Base. Als Basen kommen anorganische Basen, beispielsweise Natriumhydroxid, Kaliumhydroxid, oder organische Basen, wie Triethylamin in Frage.

Die Herstellung der Aminsalze der Hydroxyverbindungen der Formel I in denen $R_3$ = OH, erfolgt zweckmäßigerweise direkt in einem geeigneten Formulierungsmittel unter Zusatz eines Netzmittels. So wird z. B. eine 50 %ige Präparation des Triethylaminsalzes der Verbindung der Formel I, $R_1$ = 4-Cl, $R_2$ = H, $R_3$ = OH, X = Cl, hergestellt, indem man die Hydroxyverbindung (Substanz 1,3.526 g, 0,0146 Mol) in einem Glycerin (4,445g)-Rapidnetzer-Linz (0,500 g)-Gemisch suspendiert und sodann langsam Triethylamin (1,527 g, 0,01509 Mol, 3,2 % Überschuß) zutropfte. Dann wurde 2 Stunden gelinde erwärmt wobei die Temperatur von 40 °C auf 60 °C gebracht wurde. Es entstand eine homogene, 50 %ige gebrauchsfertige Mischung des Triethylaminsalzes.

Das erfindungsgemäße Verfahren eignet sich zur Bekämpfung von dikotylen aber auch monokotylen Samenunkräutern in Kulturen wie Getreide, Mais, Erdnuß, Kohlgewächsen, Kichererbsen, Tomaten und Zwiebeln.

Besonders geeignet ist das Verfahren zur Bekämpfung von

Amaranthus retoflexus
Anthemis arvensis
Centaurea arvensis
Chenopodium ficifolium
Echinochloa crus-galli
Galium aparine
Mercurialis annua
Lapsana communis
Panicum millaceum
Setaria glauca
Solanum americanum
Solanum nigrum
Stellaria media
in den oben genannten Kulturen.

Die erfindungsgemäßen Mittel können in üblicher Weise formuliert sein, beispielsweise als Lösungen, benetzbare Pulver, Emulsionskonzentrate, Emulsionen, Suspensionen, Dispersionen, Stäubemittel, Granulate u. dgl. und enthalten dann gegebenenfalls die üblichen Formulierungshilfsmittel wie Streckmittel, also flüssige Lösungsmittel, feste oder flüssige Trägerstoffe, gegebenenfalls unter Verwendung von Tensiden, also Emulgatoren und/oder Dispergier- und/oder Netzmitteln, Mahlhilfsmittel, Haftmittel und dgl. Im Fall der Verwendung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol und andere Alkylbenzole oder Alkylnaphthaline, chlorierte Aromaten oder chlorierte aliphatische Kohlenwasserstoffe, wie Chlorbenzole, Chlorethylene oder Methylenchlorid, cycloaliphatische Kohlenwasserstoffe wie Cyclohexan oder Paraffine, z. B. Erdölfraktionen, Alkohole wie Butanol oder Glykol, sowie deren Ether und Ester, Ketone, wie Aceton, Methylethylketon, Methylisobutylketon oder Cyclohexanon, stark polare Lösungsmittel wie Dimethylformamid oder Dimethylsulfoxid, sowie Wasser.

Benetzbare Pulver sind im Wasser gleichmäßig dispergierbare Präparate, die neben dem Wirkstoff außer gegebenenfalls Verdünnungs- bzw. Inertstoffen noch Netzmittel z. B.: polyoxethylierte Alkylphenole, polyoxethylierte Oleyl- oder Stearylamine, Alkyl- oder Alkylphenylsulfonate und Dispergiermittel z. B.: Na-Oleylmethyltaurid, Ligninsulfonate, oder Kondensationsprodukte von Arylsulfonaten mit Formaldehyd enthalten können.

Emulgierbare Konzentrate können beispielsweise durch Auflösen des Wirkstoffes in einem organischen Lösungsmittel unter Zusatz von einem oder mehreren Emulgatoren hergestellt werden. Als Emulgatoren kommen beispielsweise nichtionogene und ionogene Tenside, wie Polyoxyethylen-Sorbitan-Tallölester, Polyoxyethylen-Fettsäureester, Polyoxyethylen-Fettalkoholether, Alkylarylpolyglykolether, Alkylsulfonate, Alkylsulfate und Arylalkylsulfonate, sowie Eiweißhydrolysate in Frage.

Stäubemittel werden durch Vermahlen des Wirkstoffes mit fein verteilten festen Stoffen, wie natürliche Gesteinsmehle, z. B.: Kaoline, Tonerde, Talkum, Kreide, Quarz, Attapulgit, Montmorillonit oder Diatomeenerde und synthetischen Produkten wie hochdisperse Kieselsäure, Aluminiumoxid und Silikate, erhalten. Als Trägerstoffe für Granulate eignen sich beispielsweise gebrochene oder fraktionierte natürliche Gesteine, wie Calcit, Marmor, Bims, Sepiolith, Dolomit, sowie synthetische Granulate aus anorganischen oder organischen Mehlen, sowie Granulate aus organischem Material, wie Sägemehl, Kokusnußschalen, Maiskolben und Tabakstengel. In den Formulierungen können Haft- und Verdickungsmittel, wie Carboxymethylcellulose, Methylcellulose, natürliche und synthetische pulvrige, körnige und latexförmige Polymere verwendet werden, wie Gummi arabicum, Polyvinylalkohol, Polyvinylacetat.

Es können Farbstoffe, wie anorganische Pigmente, z. B.: Eisenoxid, Titanoxid, Ferrocyanblau und organische Farbstoffe, wie Alizarin, Phthalocyaninfarbstoffe verwendet werden.

Die Formulierungen enthalten im allgemeinen zwischen 0,1 und 95 Gew.% Wirkstoff, vorzugsweise zwischen 0,5 und 90 Gew.%.

Die Wirkstoffe können als solche, in Form ihrer Formulierungen oder den daraus durch weiteres Verdünnen bereiteten Anwendungsformen, wie gebrauchsfertige Lösungen, Emulsionen, Suspensionen, Pulver und Granulate angewendet werden. Die Anwendung geschieht in üblicher Weise, beispielsweise duch Gießen, Spritzen, Sprühen, Stäuben, Streuen u. dgl.

Mit den äußeren Bedingungen, wie Temperatur, Feuchtigkeit u. a. variiert die erforderliche Aufwandmenge an den erfindungsgemäßen Mitteln. Sie kann sich innerhalb weiter Grenzen bewegen und beträgt im allgemeinen zwischen 0,2 und 10 kg/ha Wirkstoffe vorzugsweise 0,3 - 1,5 kg/ha.

Beispiel 1: Herstellung der Ausgangsverbindung

3-(4'-Fluorphenyl)-pyridazinon-6

a) Aus p-Fluoracetophenon mit Glyoxylsäure und Hydrazin:

4'-Fluoracetophenon (138,1 g, 1 Mol) und Glyoxylsäuremonohydrat (27,5 g, 0,3 Mol) wurden vermischt und 1,75 Stunden bei 105 °C gerührt. Dann wurde auf 60 °C gekühlt, zwischen Wasser (500 ml) und Inhibisol (400 ml) verteilt, wobei der pH-Wert der wässrigen Phase mit $NH_4OH$-Lösung auf pH 9 gebracht wurde. Dann wurden die getrennten Schichten nochmals wechselseitig mit Inhibisol (200 ml), bzw. sehr verdünnter $NH_4OH$-Lösung (pH 9, 400 ml) gewaschen und die vereinigten wässrigen Anteile (pH ca 8,5) mit Hydrazinhydrat (16,5 g) versetzt, 4 Stunden unter Rückfluß gekocht, aufkonzentriert (400 ml Destillat), auf 0° gekühlt, das anfallende Kristallisat isoliert, mit Wasser gewaschen und zur Gewichtskonstanz getrocknet. Es wurde 3-(4'-Fluorphenyl)-pyridazinon-6 (48,0 g, 0,2524 Mol, 84,5 % d. Th.) mit Fp = 264 - 269 °C erhalten. Aus einem DMF-Ethanolgemisch umkristallisiert zeigte das Produkt einen Fp. = 268 - 270 °C.

b) Aus (4'-Fluorbenzoyl)-acrylsäure:

(4'-Fluorbenzoyl)-acrylsäure (582 g 2,998 Mol mit Fp = 134 - 137 °C) wurde in Ethanol (3300 ml) gelöst, p-Toluolsulfonsäure (23 g) und Schwefelsäure (konz., 4 ml) zugesetzt und die Mischung dann 18 Stunden unter Rückfluß gehalten. Die Mineralsäure wurde sodann durch Zusatz von wasserfreiem Natriumbicarbonat neutralisiert. Es wurde sodann von den anorganischen Salzen filtriert, zur mechanisch gerührten Lösung des Veresterungsproduktes eine Hydrazinlösung (6 Mol Hydrazin, 700 ml Wasser) zugesetzt und die Mischung drei Stunden auf Rückflußtemperatur gebracht. Es schied sich ein Festprodukt ab, das isoliert, mit Wasser gewaschen und nutschenfeucht mit verdünnter Salzsäure (700 ml HCl-konz., 1600 ml Wasser) vermischt und 2 Stunden auf Rückflußtemperatur gebracht wurde. Es wurde gekühlt, das anfallende Festprodukt isoliert, mit Wasser gewaschen und zur Gewichtskonstanz getrocknet. Man erhielt 3-(4'-Fluorphenyl)-pyridazinon-6 in Form eines gelblichen Festproduktes (390 g, 2,0508 Mol, 68,4 % d Th.) mit Fp = 266 - 269 °C.

Durch Umkristallisation aus Dioxan wurde ein fast farbloses Produkt erhalten, das ab ca. 170 °C in breite Kristalle sublimiert und bei 269 - 270,5 °C schmilzt.

c) Aus (4'-Fluorbenzoyl)alanin:

3-(4'-Fluorbenzoyl)-acrylsäure (9,7 g, 0,04996 Mol) wurde in 50 ml konzentrierter wäßriger Ammoniaklösung gelöst und die Lösung einige Tage offen abgestellt.

Durch Zugabe von Salzsäure (konz., 30 ml) wurde zunächst neutralisiert, dann weitere 20 ml konzentrierter Salzsäure und sodann Hydrazinhydrochlorid (4,1 g, 0,06 Mol) zugesetzt, worauf die Mischung 8 Stunden unter Rückfluß gekocht wurde. Nach Kühlung wurde das abgeschiedene Festprodukt isoliert, mit Wasser gewaschen und zur Gewichtskonstanz getrocknet. Man erhielt 3-(4'-Fluorphenyl)-pyridazinon-6mit Fp. = 265 - 267 °C, 8,9 g 0,04680 Mol. entsprechend einer 93,7 %igen Ausbeute d. Th..

Beispiel 2: (Vbg.Nr 68)

3-(4'-Fluorphenyl)-4,6-dichlorpyridazin

3-(4'-Fluorphenyl)-pyridazinon-6 (190 g, 0,9991 Mol) wurde mit rotem Phosphor (40 g, 1,291 Mol) vermischt, worauf man 1300 ml Phosphoroxidchlorid zusetzte. Die Suspension wurde daraufhin im Verlauf einer Stunde auf 90 °C gebracht und sodann ein mittlerer Chlorstrom in die Lösung eingeblasen. Die Temperatur der Reaktionsmischung stieg auf 100 °C und die Reaktionsmischung wurde bei dieser Temperatur gehalten. Man chlorierte 4 Stunden, vertrieb sodann die gelösten Gase durch Einleiten eines mittleren Stickstoffstromes, destillierte im Wasserstrahlvakuum 800 ml Phosphoroxidchlorid ab und zersetzte sodann auf Eis wobei man die entstehende Säure mit konzentrierten Ammoniak neutralisierte. Es resultierten 235,2 g (0,9676 Mol) an 3-(4'-Fluorphenyl)-4,6-dichlorpyridazin in Form eines Festproduktes, entsprechend einer 96,9%igen Rohausbeute. Das Produkt zeigt aus Ethanol und Cyclohexan umkristallisiert einen Schmelzpunkt von 124 - 127 °C.

Beispiel 3 (Vbg.Nr. 56):

3-(4'-Fluorphenyl)-4-hydroxy-6-chlorpyridazin

3-(4'-Fluorphenyl)-4,6-dichlorpyridazin (145,84 g, 0,600 Mol) wurde in Dioxan (800 ml) bei 60 °C gelöst, Wasser (300 ml) zugefügt und die Lösung auf Rückfluß erhitzt. Innerhalb einer halben Stunde wurde sodann Natronlauge (48 g NaOH fest, in 300 ml Wasser gelöst) zugetropft und weitere 5 Stunden unter Rückfluß gehalten. Dann wurde im Vakuum zur Trockene gebracht, der Rückstand in heißem Wasser (5 l) gelöst, von unlöslichen Anteilen filtriert und mit konzentrierter Salzsäure auf pH 1 gestellt. Der anfallende Feststoff wurde isoliert, mit Wasser gewaschen und zur weiteren Reinigung in verdünntem Ammoniak (8 l Wasser, pH 9 - 10) gelöst. Dann wurden durch vorsichtige Zugabe von Salzsäure geringe Mengen an 3-(4'-Fluorphenyl)-4-chlorpyridazinon-6 selektiv ausgefällt, abgetrennt (durch Filtration) und sodann die Hauptmenge zur Fällung gebracht (bis pH 1). Das erhaltene Festprodukt wurde isoliert, mit Wasser gewaschen und zur Gewichtskonstanz getrocknet. Es wurden 91,0 g (0,4051 Mol) an 3-(4'-Fluorphenyl)-4-hydroxy-6-chlorpyridazin mit Fp. = 254 - 260 °C (Z.P.) erhalten, entsprechend einer 67,5 %igen Ausbeute d. Th.

Beispiel 4 (Vbg.Nr. 64):

3-(4'-Fluorphenyl)-6-chlor-4-(octylthiocarbonyloxy)-pyridazin

3-(4'-Fluorphenyl)-4-hydroxy-6-chlor-pyridazin (22,5 g, 0,1 Mol) wurde in 80 ml Aceton suspendiert. Dann wurde die Lösung von 4 g (0,1 Mol) Natriumhydroxid in 80 ml Wasser zugefügt und die zunächst noch trübe Lösung auf 60 °C erwärmt. Zur nun klaren Lösung wurde Octylthiochlorameisensäureester (20,97 g, 0,1 Mol) auf einmal zugesetzt. Die Lösung erwärmte sich durch den Reaktionsvorgang und es bildete sich eine Emulsion. Es wurde 3/4 Stunden auf Rückfluß gehalten und der auf 6 abgesunkene pH-Wert mit 25 %iger Natronlauge auf 9 gestellt. Die schwerere organische Schicht des resultierten Zweiphasengemisches wurde abgetrennt und im Vakuum von den flüchtigen Anteilen befreit. Der erhaltene Rohester (braunes Öl, 36,7 g, 95 % Rohausbeute) wurde über eine Silicagelsäule gereinigt, wobei die oben angeführte Verbindung in Form eines gelben Öls ($n_D^{20}$ = 1,5567, 30,4 g, 79 % d. Th.) erhalten wurde.

Beispiel 5 (Vbg.Nr. 57):

3-(4'-Fluorphenyl)-4-(propylthiolcarbonyloxy)-6-chlorpyridazin

3-(4'-Fluorphenyl)-4-hydroxy-6-chlorpyridazin (22,4 g, 0,1 Mol) wurde in Benzol (2000 ml) durch Rühren suspendiert, Triethylamin (10,1 g, 0,1 Mol) zugefügt und die Mischung 3,5 Stunden bei Raumtemperatur gerührt. Dann wurde Propylthiolchlorkohlensäureester (13,86 g, 0,09999 Mol) innerhalb von 10 Minuten zugetropft. Es trat leichte Erwärmung ein und die Reaktionsmischung wurde weitere 3 Stunden ohne Wärmezufuhr gerührt. Vom abgeschiedenen Triethylaminhydrochlorid wurde filtriert und die benzolische Phase mit je 2 100 ml Portionen Wasser, dann sehr verdünnter Salzsäure, dann sehr verdünnter Natronlauge und sodann nochmals Wasser gewaschen. Dann wurde die organische Phase über Natriumsulfat getrocknet, mit Tierkohle geschönt und das Lösungsmittel im Vakuum, am Ende im Ölpumpenvakuum bei einer Badtemperatur von 90 °C völlig vertrieben. Es verblieben 27,0 g (0,08263 Mol) an 3-(4'-Fluorphenyl)-4-(propylthiolcarbonyloxy)-6-chlorpyridazin in Form eines gelben Öls, entsprechend einer 82,9 %igen Ausbeute d. Th..

Beispiel 6 (Vbg.Nr. 143):

3-(4'-Bromphenyl)-4-(2,2-dimethylpropyloxycarbonyloxy)-6-chlorpyridazin

3-(4'-Bromphenyl)-4-hydroxy-6-chlorpyridazin (erhalten analog den Beispielen 1 - 3) (8,57 g, 0,03 Mol) wurde in Benzol (90 ml) suspendiert und Triethylamin (3,19 g) zugefügt. Es trat eine sichtbare Veränderung des Feststoffes ein. Es wurde 30 Minuten gerührt und sodann eine Lösung von Chlorameisensäure-2,2-dimethylpropylester (4,38 g, 0,0291 Mol) in 20 ml Benzol in 30 Minuten zugetropft. Die Mischung erwärmte sich leicht. Nach drei Stunden wurden 20 ml Wasser zugesetzt, 5 Minuten kräftig gerührt und sodann die benzolische Phase abgetrennt, über Natriumsulfat getrocknet und das Lösungsmittel im Vakuum entfernt. Der verbleibende Rückstand erstarrte und wurde zweimal aus Diisopropylether umkristallisiert. Es wurde 3-(4'-Bromphenyl)-4-(2,2-dimethylpropyloxycarbonyloxy)-6-chlorpyridazin in Form farbloser Kristallprismen mit Fp. = 114 - 116 °C erhalten. (6,7 g, 0,01676 Mol, 57,6 % d. Th.)

Beispiel 7 (Vbg.Nr. 19):

3-(4'-Chlorphenyl)-4,6-dibrompyridazin

3-(4'-Chlorphenyl)-4-hydroxy-6-chlorpyridazin (Verbindung 1, 24,1 g, 0,09997 Mol) wurde im Reaktionskolben vorgelegt, eine Lösung von Phosphoroxidbromid (85,8 g 0,3 Mol) in Toluol (150 ml) zugefügt worauf das Reaktionsgemisch eine Stunde bei Zimmertemperatur gerührt wurde. Dann wurde drei Stunden zum leichten Rückfluß erwärmt, wobei Bromwasserstoffgas abgegeben wurde. Man zersetzte auf Eis, wodurch ein teigiges Reaktionsprodukt erhalten wurde das beim Anreiben mit Petrolether erstarrte. Das Produkt wurde noch gut mit Wasser gewaschen, isoliert und zur Gewichtskonstanz getrocknet. Es wurden 30,0 g (0,0861 Mol, 86,1 % d. Th.) an 3-(4'-Chlorphenyl)-4,6-dibrompyridazin mit Fp. = 160 - 162 °C erhalten. Aus einem Ethanol-Dioxan Gemisch (20 + 3) umkristallisiert zeigt die Probe Fp. = 163 - 165 °C.

Beispiel 8 (Vbg.Nr. 16):

3-(4'-Chlorphenyl)-4-hydroxy-6-brompyridazin

3-(4'-Chlorphenyl)-4,6-dibrompyridazin (52,2 g, 0,1498 Mol) wurde in Dioxan (300 ml) heiß gelöst und bei leichtem Rückfluß eine Lösung von 12 g (0,3 Mol) Natronlauge in 50 ml Wasser innerhalb von 10 Minuten zugetropft. Es wurde weitere 4,5 Stunden unter Rückfluß gehalten, das Dioxan im Vakuum abgedampft und der feste Rückstand in 1 l Wasser heiß (95°C) gelöst. Mit Hilfe von Tierkohle wurde heiß von wenig Festprodukt (teerig) filtriert und das Hydrolyseprodukt mit Salzsäure gefällt. Das isolierte Produkt (Feststoff) wurde nutschenfeucht in 1 l Wasser aufgeschlämmt worauf der pH-Wert der Suspension bei 65°C mit Ammoniak auf 9 - 10 gebracht wurde. Der größere Teil des Produktes ging in Lösung, die unlöslichen Anteile wurden isoliert und nochmals mit Ammoniak behandelt. Die vereinigten Filtrate wurden sodann mit Salzsäure angesäuert, das anfallende 3-(4'-Chlorphenyl)-4-hydroxy-6-brompyridazin isoliert, mit Wasser gewaschen und zur Gewichtskonstanz getrocknet. Dabei wurden 27,5 g (0,09631 Mol), das sind 64,3 % d. Th., Fp. = 262 - 264°C erhalten.

Beispiel 9 (Vbg.Nr. 17):

3-(4'-Chlorphenyl)-4-(butylthiocarbonyloxy)-6-brompyridazin

3-(4'-Chlorphenyl)-4-hydroxy-6-brompyridazin (14,2 g, 0,0497 Mol) wurde in 100 ml Benzol suspendiert, Triethylamin (5,5 g, 0,0543 Mol) zugegeben und das Gemisch zur Vervollständigung der Reaktion 3,5 Stunden bei Zimmertemperatur gerührt. Dann wurde S-Butylthiochlorkohlensäureester (7,85 g, 0,05162 Mol) innerhalb von 5 Minuten in die Mischung eingetropft und das Reaktionsgemisch weitere 3 Stunden ohne Wärmezufuhr gerührt. Vom ausgeschiedenen Triethylaminhydrochlorid wurde filtriert und die benzolische Lösung nacheinander mit je 150 ml Portionen Wasser, sehr stark verdünnter Salzsäure, sehr stark verdünnter Natronlauge dann wieder Wasser gewaschen und über Natriumsulfat getrocknet. Das Lösungsmittel wurde sodann im Vakuum, gegen Ende im Ölpumpenvakuum (0,2 Torr, 90°C Badtemperatur) völlig entfernt. Das zunächst ölig anfallende S-Butylthiocarbonat des 3-(4'-chlorphenyl)-4-hydroxy-6-chlorpyridazin, 17,5 g, 0,04356 Mol, 87,6 % d. Th., erstarrte bei längerem Stehen und zeigte aus Hexan umgelöst Fp. = 68 - 71°C.

Beispiel 10:

3-(3'-Nitro-4'chlorphenyl)-4-hydroxy-6-chlorpyridazin (Vbg. 138)

3-(4'-Chlorphenyl)-4-hydroxy-6-chlorpyridazin (12 g, 0,04978 Mol) wurde innerhalb von 15 Minuten bei einer Temperatur von 0 - 5°C in gekühlte hochkonzentrierte Salpetersäure eingetragen. Es wurde weitere 30 Minuten bei 0°C gerührt und sodann das Reaktionsgemisch in Eiswasser eingerührt. Das anfallende gelbe Festprodukt wurde isoliert, mit Wasser gewaschen und mit $NH_4OH/HCl$ umgefällt. Nach Trocknung wurden 11 g, 0,0385 Mol an 3-(3'-Nitro-4'-chlorphenyl)-4-hydroxy-6-chlorpyridazin mit Fp 204 - 209°C erhalten, entsprechend einer 77,25 %igen Rohausbeute. Die Probe erwies sich laut DC-Analyse ($SiO_2$/10 % Ethanol-Chloroform) als weitgehend einheitlich. Aus Ethanol umkristallisiert schmilzt die Verbindung bei 208 - 211°C.

3-(3'-Nitro-4'-chlorphenyl)-4-butylthiocarbonyloxy)-6-chlorpyridazin (Vbg.Nr.88)

3-(3'-Nitro-4'-chlorphenyl)4-hydroxy-6-chlorpyridazin (14,3 g, 0,05 Mol) wurde in Benzol nach üblicher Methode mit Butylthiochlorameisensäureester (7,88 g) und mit Triethylamin (5,05 g) als Salzsäurebinder umgesetzt. Es wurden 3-(3'-Nitro-4'-chlorphenyl)-4-(butylthiocarbonyloxy)-6-chlorpyridazin in Form eines hellbraunen Öls erhalten. 17.0 g, 0,04226 Mol, 84,55 % d. Th., $n_D^{20}$ = 1,6073

Beispiele:

| Vbg. | $R_1$ | $R_2$ | n | m | $R_3$ | X | Fp(°C)/ $n_D^{20}$ |
|---|---|---|---|---|---|---|---|
| 1 | 4-Cl | - | 1 | - | OH | Cl | 264-268* |
| 2 | 4-Cl | - | 1 | - | $OC(O)C_7H_{15}$-n | Cl | 40-43,5 |
| 3 | 4-Cl | - | 1 | - | $OC(O)N(CH_3)_2$ | Cl | 70-82 |
| 4 | 4-Cl | - | 1 | - | $OC(O)OCH_2CH(CH_3)_2$ | Cl | 72-74 |
| 5 | 4-Cl | - | 1 | - | $O(CO)OC_5H_{11}$-n | Cl | |
| 6 | 4-Cl | - | 1 | - | $OC(O)SC_3H_7$-n | Cl | 39-45 |
| 7 | 4-Cl | - | 1 | - | $OC(O)SC_4H_9$-n | Cl | 77,5-80 |
| 8 | 4-Cl | - | 1 | - | $OC(O)SC_4H_9$-t | Cl | 120-123 |
| 9 | 4-Cl | - | 1 | - | $OC(O)SC_5H_{11}$-n | Cl | 32-38 |
| 10 | 4-Cl | - | 1 | - | $OC(O)SC_6H_{13}$-n | Cl | |
| 11 | 4-Cl | - | 1 | - | $OC(O)SC_7H_{15}$-n | Cl | |
| 12 | 4-Cl | - | 1 | - | $OC(O)SC_8H_{17}$-n | Cl | 1,5733 |
| 13 | 4-Cl | - | 1 | - | $OC(O)SC_{12}H_{25}$-n | Cl | |
| 14 | 4-Cl | - | 1 | - | $OC(O)SC_6H_{11}$-c | Cl | |
| 15 | 4-Cl | - | 1 | - | $OC(O)SC_6H_5$ | Cl | 105-108 |
| 16 | 4-Cl | - | 1 | - | OH | Br | 262-264 |
| 17 | 4-Cl | - | 1 | - | $OC(O)SC_4H_9$-n | Br | 68-71 |
| 18 | 4-Cl | - | 1 | - | $OC(O)SC_8H_{17}$-n | Br | |
| 19 | 4-Cl | - | 1 | - | Br | Br | 163-165 |
| 20 | 4-Br | - | 1 | - | OH | Cl | 271-276 |
| 21 | 4-Br | - | 1 | - | $OC(O)(CH_2)_5CH_3$ | Cl | |
| 22 | 4-Br | - | 1 | - | $OC(O)CH_2CH(CH_3)_2$ | Cl | 49-56 |
| 23 | 4-Br | - | 1 | - | $OC(O)C_5H_{11}$-n | Cl | |
| 24 | 4-Br | - | 1 | - | $OC(O)SC_3H_7$-n | Cl | 75-77 |
| 25 | 4-Br | - | 1 | - | $OC(O)SC_4H_9$-sec | Cl | 71-73 |
| 26 | 4-Br | - | 1 | - | $OC(O)SC_4H_9$-t | Cl | 121-124 |
| 27 | 4-Br | - | 1 | - | $OC(O)SC_5H_{11}$-n | Cl | 57-59 |
| 28 | 4-Br | - | 1 | - | $OC(O)SC_5H_{11}$-i | Cl | 72-74 |
| 29 | 4-Br | - | 1 | - | $OC(O)SC_5H_{11}$-t | Cl | 75-88 |
| 30 | 4-Br | - | 1 | - | $OC(O)SC_7H_{15}$-n | Cl | 63-65 |
| 31 | 4-Br | - | 1 | - | $OC(O)SC_8H_{17}$-n | Cl | 45-47 |
| 32 | 4-Br | - | 1 | - | $OC(O)SC_{10}H_{21}$-n | Cl | 51-54 |
| 33 | 4-Br | - | 1 | - | $OC(O)SC_6H_{11}$-c | Cl | 69-73 |
| 34 | 3-Br | - | 1 | - | Cl | Cl | 112-114 |

| | | | | | | | |
|---|---|---|---|---|---|---|---|
| 35 | 3-Br | - | 1 | - | OH | Cl | 208-212 |
| 36 | 3-Br | - | 1 | - | $OC(O)CH(C_2H_5)C_4H_9$ | Cl | 1,5680($n_D^{25}$) |
| 37 | 3-Br | - | 1 | - | $OC(O)OC_4H_9$-i | Cl | 1,5795($n_D^{25}$) |
| 38 | 3-Br | - | 1 | - | $OC(O)SC_4H_9$-n | Cl | 1,6080($n_D^{25}$) |
| 39 | 3-Br | - | 1 | - | $OC(O)SC_4H_9$-S | Cl | 1,6085($n_D^{25}$) |
| 40 | 3-Br | - | 1 | - | $OC(O)SC_8H_{17}$-n | Cl | 1,5792($n_D^{25}$) |
| 41 | 4-$CH_3O$ | - | 1 | - | OH | Cl | 245-247 |
| 42 | 4-$CH_3O$ | - | 1 | - | $OC(O)C_6H_{13}$-n | Cl | 1,5598 |
| 43 | 4-$CH_3O$ | - | 1 | - | $OC(O)C_7H_{15}$-n | Cl | 1,5539 |
| 44 | 4-$CH_3O$ | - | 1 | - | $OC(O)OC_4H_9$-i | Cl | 58-59,5 |
| 45 | 4-$CH_3O$ | - | 1 | - | $OC(O)OC_5H_{11}$-n | Cl | 1,5677 |
| 46 | 4-$CH_3O$ | - | 1 | - | $(OC(O)SC_4H_9$-n | Cl | 44-53 |
| 47 | 4-$CH_3O$ | - | 1 | - | Cl | Cl | 144-147 |
| 48 | 3-$CF_3$ | - | 1 | - | OH | Cl | 238-240* |
| 49 | 3-$CF_3$ | - | 1 | - | $OC(O)(CH_2)_5CH_3$ | Cl | 1,5162 |
| 50 | 3-$CF_3$ | - | 1 | - | $OC(O)(CH_2)_6CH_3$ | Cl | 1,5145 |
| 51 | 3-$CF_3$ | - | 1 | - | $OC(O)OC_4H_9$-i | Cl | 45-48,5 |
| 52 | 3-$CF_3$ | - | 1 | - | $OC(O)OC_5H_{11}$-n | Cl | 1,5161 |
| 53 | 3-$CF_3$ | - | 1 | - | $OC(O)SC_4H_9$-n | Cl | 36-40 |
| 54 | 3-$CF_3$ | - | 1 | - | $OC(O)SC_8H_{17}$-n | Cl | 1,5323 |
| 55 | 3-$CF_3$ | - | 1 | - | Cl | Cl | 80-81,5 |
| 56 | 4-F | - | 1 | - | OH | Cl | 254-260* |
| 57 | 4-F | - | 1 | - | $OC(O)SC_3H_7$-n | Cl | |
| 58 | 4-F | - | 1 | - | $OC(O)SC_4H_9$ | Cl | |
| 59 | 4-F | - | 1 | - | $OC(O)SC_4H_9$-s | Cl | |
| 60 | 4-F | - | 1 | - | $OC(O)SC_4H_9$-t | Cl | 109-111 |
| 61 | 4-F | - | 1 | - | $OC(O)SC_5H_{11}$-i | Cl | |
| 62 | 4-F | - | 1 | - | $OC(O)OC_5H_{11}$-t | Cl | 65-68 |
| 63 | 4-F | - | 1 | - | $OC(O)SC_7H_{15}$-n | Cl | |
| 64 | 4-F | - | 1 | - | $OC(O)SC_8H_{17}$-n | Cl | 1,5567 |
| 65 | 4-F | - | 1 | - | $OC(O)SC_{12}H_{25}$-n | Cl | 33-36 |
| 66 | 4-F | - | 1 | - | $OC(O)SC_6H_{11}$-c | Cl | 57-59 |
| 67 | 4-$C_2H_5$ | - | 1 | - | OH | Cl | 224-225 |
| 68 | 4-F | - | 1 | - | Cl | Cl | 124-127 |
| 69 | 4-Cl | 2-Cl | 1 | 1 | OH | Cl | 233-237* |
| 70 | 4-Cl | 2-Cl | 1 | 1 | $OC(O)SC_4H_9$-n | Cl | |
| 71 | 4-Cl | 2-Cl | 1 | 1 | Cl | Cl | 144-147 |
| 72 | 4-$C_2H_5$ | - | 1 | - | $OC(O)SC_8H_{17}$ | Cl | 1,5637 |

| | | | | | | | |
|---|---|---|---|---|---|---|---|
| 73 | 4-Cl | 3-Cl | 1 | 1 | $OC(O)OCH_2C(CH_3)_3$ | Cl | 109-111,5 |
| 74 | 4-Cl | $3NO_2$ | 1 | 1 | $OC(O)SC_8H_{17}$-n | Cl | 1,5821 |
| 75 | 4-Br | $3NO_2$ | 1 | 1 | OH | Cl | 209-212 |
| 76 | $4-C_2H_5$ | - | 1 | - | $OC(O)SC_4H_9$-n | Cl | |
| 77 | 4-Cl | 3-Cl | 1 | 1 | OH | Cl | 239-243 |
| 78 | 4-Cl | 3-Cl | 1 | 1 | $OC(O)CH_2CH(CH_3)_2$ | Cl | 48-52 |
| 79 | 4-Cl | 3-Cl | 1 | 1 | $OC(O)SC_3H_7$-n | Cl | 1,6186 |
| 80 | 4-Cl | 3-Cl | 1 | 1 | $OC(O)SC_4H_9$-n | Cl | 1,6108 |
| 81 | 4-Cl | 3-Cl | 1 | 1 | $OC(O)SC_8H_{17}$-n | Cl | 1,5814 |
| 82 | 4-Br | $3NO_2$ | 1 | 1 | $OC(O)SC_4H_9$ | Cl | 1,6199 |
| 83 | $4-CH_3$ | - | 1 | - | OH | Cl | 240* |
| 84 | $4-CH_3$ | - | 1 | - | $OC(O)SC_4H_9$-n | Cl | |
| 85 | $4-CH_3$ | $3CH_3$ | 1 | 1 | $OC(O)OC_4H_9$-i | Cl | |
| 86 | $4-CH_3S$ | - | 1 | - | $OC(O)OC_5H_{11}$-n | Cl | 65-66,5 |
| 87 | $4-CH_3S$ | - | 1 | - | $OC(O)SC_4H_9$-n | Cl | 1,6332 |
| 88 | 4-Cl | $3NO_2$ | 1 | 1 | $OC(O)SC_4H_9$-n | Cl | 1,6073 |
| 89 | 4-Br | $3NO_2$ | 1 | 1 | $OC(O)OC_7H_{15}$-n | Cl | 69-72 |
| 90 | 4-Br | $3NO_2$ | 1 | 1 | $OC(O)OC_4H_9$-i | Cl | 1,5942 |
| 91 | $4-CH_3$ | $2CH_3$ | 1 | 1 | Cl | Cl | 92-100 |
| 92 | $4(5)NO_2$ | $2(3)NO_2$ | 1 | 1 | $OC(O)OCH_2CH(CH_3)_2$ | Cl | - |
| 93 | 4-Cl,3-Cl | 2-Cl | 2 | 1 | OH | Cl | 230-237 |
| 94 | 4-Cl,3-Cl | 2-Cl | 2 | 1 | $OC(O)SC_4H_9$-n | Cl | |
| 95 | $4-C_4H_9O$ | - | 1 | - | $OC(O)(CH_2)_5CH_3$ | Cl | |
| 96 | $4-CH_3O$ | $3NO_2$ | 1 | 1 | Cl | Cl | 213-215 |
| 97 | $4-C_6H_5O$ | - | 1 | - | OH | Cl | 197-201* |
| 98 | $4-C_6H_5O$ | - | 1 | - | $OC(O)OC_5H_{11}$-n | Cl | 1,5901 |
| 99 | $4-C_6H_5O$ | - | 1 | - | $OC(O)SC_4H_9$-n | Cl | 1,6179 |
| 100 | 4- Br | $3NO_2$ | 1 | 1 | $OC(O)SC_8H_{17}$-n | Cl | 1,5895 |
| 101 | 5-Cl | 2-Cl | 1 | 1 | $OC(O)SC_4H_9$-n | Cl | |
| 102 | 4-F | - | 1 | - | $OC(O)SC_5H_{11}$-n | Cl | |
| 103 | 4-Br | $3NO_2$ | 1 | 1 | $OC(O)CH(C_2H_5)C_4H_9$ | Cl | 69-72 |
| 104 | $4-CH_3S$ | - | 1 | - | OH | Cl | 230-231 |
| 105 | $4-CH_3SO_2$ | - | 1 | - | OH | Cl | 203-212 |
| 106 | $4-CH_3S$ | - | 1 | - | Cl | Cl | 120-121 |
| 107 | $2-CH_3$ | $5CH_3$ | 1 | 1 | Cl | Cl | 100-101 |
| 108 | $4(5)NO_2$ | $2(3)NO_2$ | 1 | 1 | OH | Cl | 250-252 |
| 109 | $4(5)NO_2$ | $2(3)NO_2$ | 1 | 1 | $OC(O)OC_5H_{11}$ | Cl | |
| 110 | $4(5)NO_2$ | $2(3)NO_2$ | 1 | 1 | $OC(O)N(CH_3)_2$ | Cl | |

| | | | | | | | |
|---|---|---|---|---|---|---|---|
| 111 | 4Cl,3Cl | 2Cl | 2 | 1 | Cl | Cl | 115-118 |
| 112 | 4-$C_4H_9O$ | - | 1 | - | OC(O)S$C_6H_{11}$-c | Cl | 1,5882 |
| 113 | 4-$C_4H_9O$ | - | 1 | - | OC(O)S$C_8H_{17}$-n | Cl | 50-53,5 |
| 114 | 4-$C_4H_9O$ | - | 1 | - | OC(O)S$C_{12}H_{25}$-n | Cl | 50-56 |
| 115 | 4-$C_4H_9O$ | - | 1 | - | OC(O)N$(CH_3)_2$ | Cl | 97,5-99 |
| 116 | 4-$C_4H_9O$ | - | 1 | - | Cl | Cl | 62-63 |
| 117 | 4-$CH_3O$ | 3$NO_2$ | 1 | 1 | OH | Cl | 250-254* |
| 118 | 4-$C_6H_5O$ | - | 1 | - | Cl | Cl | 129-133 |
| 119 | 3-$NH_2$ | - | 1 | - | OH | Cl | 185-188 |
| 120 | 4-$C_6H_{11}$-c | - | 1 | - | OH | Cl | 225-235 |
| 121 | 3-$CH_3$ | 4$CH_3$ | 1 | 1 | OH | Cl | 222-224 |
| 122 | 4-$C_2H_5$ | - | 1 | - | OC(O)O$CH_2C(CH_3)_3$ | Cl | 70-71,5 |
| 123 | 3-$CH_3$ | 4$CH_3$ | 1 | 1 | OC(O)$C_5H_{11}$-n | Cl | |
| 124 | 3-$CH_3$ | 4$CH_3$ | 1 | 1 | Cl | Cl | 90-92 |
| 125 | 3-Cl | 4Cl | 1 | 1 | Cl | Cl | 150-152 |
| 126 | 4-$CH_3$-$SO_2$ | - | 1 | - | OC(O)S$C_4H_9$-n | Cl | 99-102 |
| 127 | 5-$CH_3$ | 2$CH_3$ | 1 | 1 | OH | Cl | 163-170 |
| 128 | 4(5)$NO_2$ | 2(3)$NO_2$ | 1 | 1 | Cl | Cl | 161-166 |
| 129 | 4Cl,3Cl | 2Cl | 2 | 1 | OC(O)S$C_8H_{17}$-n | Cl | |
| 130 | 4-$C_4H_9O$ | - | 1 | - | OH | Cl | 216-218 |
| 131 | 4-$C_4H_9O$ | - | 1 | - | OC(O)O$C_5H_{11}$-n | Cl | 37-39 |
| 132 | 4-$C_4H_9O$ | - | 1 | - | OC(O)O$C_4H_9$-i | Cl | 68-70 |
| 133 | 4-$C_4H_9O$ | - | 1 | - | OC(O)S$C_4H_9$-n | Cl | 52-54 |
| 134 | 3$NO_2$5$NO_2$ | 4$CH_3O$ | 2 | 1 | OH | Cl | 280 |
| 135 | 4-$C_6H_{11}$-c | - | 1 | - | OC(O)S$C_4H_9$-n | Cl | 1,5878 |
| 136 | 4-$C_6H_{11}$-c | - | 1 | - | Cl | Cl | 115-118 |
| 137 | 4Cl | - | 1 | - | OC(O)S$CH_2CH_2C_6H_5$ | Cl | 1,6303 |
| 138 | 4-Cl | 3$NO_2$ | 1 | 1 | OH | Cl | 208-211 |
| 139 | 3-$NO_2$ | - | 1 | - | Cl | Cl | 186-191 |
| 140 | 3-$NO_2$ | - | 1 | - | OH | Cl | 246-249 |
| 141 | 3-$NO_2$ | - | 1 | - | OC(O)O$C_5H_{11}$-n | Cl | |
| 142 | 4-OH | - | 1 | - | OH | Cl | 280* |
| 143 | 4-Br | - | 1 | - | OC(O)O$CH_2C(CH_3)_3$ | Cl | 114-116 |
| 144 | 4-CN | - | 1 | - | Cl | Cl | 210-222 |
| 145 | 4-CN | - | 1 | - | OH | Cl | 286-290 |

*Zersetzung

Beispiel A:

20 Teile der Verbindung Nr. 9 werden geschmolzen und in flüssigem Zustand mit 20 Teilen gefällter, gemahlener Kieselsäure, 50 Teilen Kieselgur, 3 Teilen des Na-Salzes der Diisobutyl-Naphthalinsulfonsäure

und 7 Teile Na-Ligninsulfonat innig vermischt und in einer Planetenkugelmühle 1 Stunde vermahlen. Man erhält ein Spritzpulver, das leicht in Wasser dispergiert und daher zur Herstellung einer biologisch aktiven Spritzbrühe geeignet ist.

Beispiel B:

40 Teile der Verbindung Nr. 57, einem hellbraunen Öl, werden mit 40 Teilen gefällter, gemahlener Kieselsäure, 10 Teilen Kieselgur, 4 Teilen Kreide, 3 Teilen des Na-Salzes der Diisobutyl-Naphthalinsulfonsäure und 3 Teilen Na-N-Oleoyl-N-methyltaurid innig vermischt und in einer Planetenkugelmühle 1 Stunde vermahlen. Man erhält ein Spritzpulver, das leicht in Wasser dispergiert und daher zur Herstellung einer biologisch aktiven Spritzbrühe geeignet ist.

Beispiel C:

10 Teile der Verbindung Nr. 19 werden mit 10 Teilen gefällter, gemahlener Kieselsäure, 70 Teilen Attaclay, 2 Teilen des Na-Salzes der Diisobutyl-Naphthalinsulfonsäure, 1 Teil Na-N-Oleoyl-N-methyltaurid und 7 Teilen Na-Ligninsulfonat innig vermischt und in einer Planetenkugelmühle 1 Stunde lang vermahlen. Man erhält ein Spritzpulver, das leicht in Wasser dispergiert und daher zur Herstellung einer biologisch aktiven Spritzbrühe geeignet ist.

Beispiel D:

50 Teile der Verbindung Nr. 36 werden mit 45 Teilen gefällter, gemahlener Kieselsäure, 2 Teilen des Na-Salzes der Diisobutyl-Naphthalinsulfonsäure und 3 Teilen Na-N-Oleoyl-N-methyltaurid innig vermischt und in einer Planetenkugelmühle 1 Stunde lang vermahlen. Man erhält ein Spritzpulver, das leicht in Wasser dispergiert und daher zur Herstellung einer biologisch aktiven Spritzbrühe geeignet ist.

Beispiel E:

40 Teile der Verbindung Nr. 64 werden mit 45 Teilen gefällter, gemahlener Kieselsäure, 9 Teilen fein gemahlener Kreide, 3 Teilen des Na-Salzes der Diisobutyl-Naphthalinsulfonsäure und 3 Teilen Na-N-Oleoyl-N-methyltaurid innig vermischt und in einer Planetenkugelmühle 1 Stunde lang vermahlen. Man erhält ein Spritzpulver, das leicht in Wasser dispergiert und daher zur Herstellung einer biologisch aktiven Spritzbrühe geeignet ist.

Beispiel F:

25 Teile der Verbindung Nr. 53 werden in 65 Teilen Solvesso 100 (aromatisches Lösungsmittel, bestehend hauptsächlich aus Trimethylbenzolen) gelöst und 10 Teile einer Emulgatormischung, bestehend aus Calcium-dodecylbenzolsulfonat und einem Polyoxy-ethylen-Sorbitan-Tallölester zugegeben. Man erhält ein emulgierbares Konzentrat, das sich zur Herstellung einer biologisch aktiven Spritzbrühe eignet.

Beispiel G:

60 Teile der Verbindung Nr. 52 werden in einem Gemisch aus 20 Teilen Xylol und 10 Teilen Butyrolacton gelöst und 10 Teile einer Emulgatormischung, bestehend aus Calzium-dodecylbenzol-sulfonat und einem Polyoxyethylen-Sorbitan-Tallölester zugegeben. Man erhält ein emulgierbares Konzentrat, das sich zur Herstellung einer biologisch aktiven Spritzbrühe eignet.

Beispiel H:

40 Teile der Verbindung Nr. 64 werden in einem Gemisch aus 30 Teilen Solvesso 100 und 20 Teilen Cyclohexanon gelöst und 10 Teile einer Emulgatormischung, bestehend aus Calciumdodecylbenzolsulfonat zolsulfonat und einem polyethoxylierten Ricinusöl zugegeben. Man erhält ein emulgierbares Konzentrat, das sich zur Herstellung einer biologisch aktiven Spritzbrühe eignet.

Beispiel I:

10 Teile der Verbindung Nr. 22 werden in 80 Teilen Solvesso 100 gelöst und 10 Teile einer Emulgatormischung, bestehend aus Calzium-dodecylbenzolsulfonat und einem polyethoxylierten Nonylphenol zugegeben. Man erhält ein emulgierbares Konzentrat, das sich zur Herstellung einer biologisch aktiven Spritzbrühe eignet.

Beispiel J: Herbizide Wirkung

Die Applikation einer bestimmten Wirkstoffmenge (siehe jeweilige Testansätze) wurden im Nachauflaufverfahren (3 - 5 Blattstadium der Testpflanzen) durchgeführt.
Die Auswertung der Versuche erfolgte nach dem Boniturschema der Tabelle 1.
Die Versuche wurden 2 - 3 mal optisch bonitiert (Skala 1 - 9). Die dargestellten Werte sind Durchschnittswerte aller Beurteilungen. Die im folgenden verwendeten Codes der Testpflanzen sind in Tab. 2 aufgeschlüsselt.

Tabelle 1

| EWRC - Skala 1 - 9: | | |
|---|---|---|
| Note | Herbizide Wirkung | in % |
| 1 | ausgezeichnet | 100 |
| 2 | sehr gut | 97,5 |
| 3 | gut | 95,0 |
| 4 | befriedigend | 90,0 |
| 5 | noch ausreichend | 85,0 |
| 6 | nicht ausreichend | 80,0 |
| 7 | gering | 75,0 |
| 8 | sehr gering | 65,0 |
| 9 | keine Wirkung | 32,5 |

Tabelle 2

| Codes der verwendeten Testpflanzen | | |
|---|---|---|
| Code | lat. Name | dt. Name |
| AMARE | Amaranthus retofluxus | zurückgekrümmter Amarant |
| ANTAR | Anthemis arvensis | Acker - Hundskamille |
| CENAR | Centaurea arvensis | Kornblume |
| CHEFI | Chenopodium ficifolium | Feigenblättriger Gänsefuß |
| ECHCG | Echinochloa crus-galli | Hühnerhirse |
| GALAP | Galium aparine | Klettenlabkraut |
| MERAN | Mercurialis annua | Einjähriges Bingelkraut |
| LAPCO | Lapsana communis | Rainkohl |
| PANMI | Panicum milliaceum | Kulturhirse |
| SETGL | Setaria glauca | Gelbe Borstenhirse |
| SOLAM | Solanum americanum | Amerikanischer Nachtschatten |
| SOLNI | Solanum nigrum | Schwarzer Nachtschatten |
| STEME | Stellaria media | Vogelmiere |

Standard: 6-Chlor-3-phenylpyridazin-4yl-5-octylthiocarbonat

| AS: aktive Substanz | a) Nopon (Ölzusatz) |
|---|---|

Beispiel K:

| Verbindung | Dosierung g AS/ha | Herbizide Wirkung (Skala 1 - 9) | | | | |
|---|---|---|---|---|---|---|
| | | GALAP | STEME | LAPCO | ANTAR | ø |
| Standard | 300 | 1,7 | 4,3 | 1,7 | 1,7 | 2,35 |
| Standard + a | 300 + 5,0 l | 1,7 | 5,0 | 1,7 | 1,3 | 2,42 |
| Verb.64 | 300 | 1,3 | 3,3 | 1,7 | 1,3 | 1,9 |
| Verb.64 + a | 300 + 5,0 l | 1,0 | 2,3 | 1,7 | 1,3 | 1,58 |

Die in diesem Beispiel angegebene Verbindung übertrifft in obigem Versuch den vorgelegten Standard. Von analogen Verbindungen wurden ähnliche Wirkprofile sichergestellt.

Beispiel L

| Verbindung | Dosierung g AS/ha | Herbizide Wirkung (Skala 1 - 9) | | | | |
|---|---|---|---|---|---|---|
| | | SOLNI | CHEFI | MERAN | AMARE | ø |
| Stan dard Verb.64 | 300 | 1,0 | 1,0 | 3,0 | 1,5 | 1,63 |
| | 150 | 1,0 | 1,0 | 4,0 | 2,0 | 2,0 |
| | 300 | 1,0 | 1,0 | 2,0 | 1,5 | 1,38 |
| | 150 | 1,0 | 1,0 | 2,0 | 1,5 | 1,38 |

Von analogen Verbindungen wurden ähnliche Werte erreicht.

Beispiel M:

| Verb. | Produkt kg/ha | Herbizide Wirkung (Skala 1 - 9) | | | | |
|---|---|---|---|---|---|---|
| | | GALAP | STEME | LAPCO | ANTAR | ø |
| Standard Verb.64 | 0,6 | 5,0 | 7,0 | 1,0 | 3,5 | 4,13 |
| | 0,4 | 5,0 | 7,0 | 3,5 | 6,0 | 5,38 |
| | 0,6 | 1,0 | 4,5 | 1,0 | 1,0 | 1,8 |
| | 0,4 | 1,0 | 5,0 | 1,0 | 2,5 | 2,38 |

Von analogen Verbindungen wurden ähnliche Werte erreicht.

Beispiel N:

Herbizide Wirksamkeit gegen Solanum americanum (SOLAM) = Amerikanischer Nachtschatten.

| Verbindung | Dosierung g AS/ha | Herbizide Wirkung (Skala 1 - 9) SOLAM |
|---|---|---|
| Verbindung 64 | 300 | 2,7 |
| | 200 | 5,7 |
| | 100 | 6,7 |

Beispiel O:

Herbizide Wirkung gegen hirseartige Gräser

| Verbindung | Produkt kg/ha | Herbizide (%) Wirkung | | | |
|---|---|---|---|---|---|
| | | ECHGG | SETGL | PANMI | ø |
| Standard Verb.64 | 0,5<br>0,75<br>1,125<br>1,688 | 10,3<br>26,9<br>64,7<br>72,1 | 44,2<br>44,3<br>49,5<br>63,5 | 0<br>0<br>26,7<br>65,6 | 18,17<br>23,7<br>46,97<br>67,1 |

In allen Fällen werden von Verbindung 64 die Werte des Standard übertroffen.

Beispiel P:

| Verbindung | Dosierung g AS/ha | Herbizide Wirkung (Skala 1 - 9) | | | | |
|---|---|---|---|---|---|---|
| | | SOLNI | CHEFI | MERAN | AMARE | ø |
| Standard | 300 | 1,3 | 2,9 | 1,3 | 2,4 | 1,98 |
| Verb. 57 | 300 | 1,0 | 3,1 | 1,0 | 1,1 | 1,55 |
| Verb. 59 | 300 | 1,0 | 2,9 | 1,0 | 1,0 | 1,48 |
| Verb.100 | 300 | 1,0 | 2,9 | 1,3 | 1,1 | 1,58 |
| Verb. 62 | 300 | 1,0 | 3,9 | 1,0 | 2,4 | 2,1 |
| Verb. 63 | 300 | 1,0 | 3,0 | 1,0 | 2,6 | 1,9 |
| Verb.64 | 300 | 1,0 | 2,7 | 1,1 | 2,7 | 1,88 |

Beispiel Q:

| Verbindung | Dosierung g AS/ha | Herbizide Wirkung (Skala 1 -9) | | | | |
|---|---|---|---|---|---|---|
| | | ANTAR | CENAR | LAPCO | GALAP | ø |
| Standard | 300 | 1,3 | 2,2 | 1,1 | 2,1 | 1,68 |
| Verb. 9 | 300 | 1,0 | 2,7 | 1,0 | 2,0 | 1,68 |
| Verb. 10 | 300 | 1,0 | 2,6 | 1,0 | 2,1 | 1,68 |
| Verb. 11 | 300 | 1,0 | 2,6 | 1,0 | 2,0 | 1,65 |

Beispiel R:

| Verbindung | Dosierung g/AS/h | Herbizide Wirkung (Skala 1 - 9) | | | | |
|---|---|---|---|---|---|---|
| | | ANTAR | CENAR | LAPCO | GALAP | ø |
| Standard | 200 | 1,0 | 2,4 | 1,7 | 2,0 | 1,78 |
| Verb. 5 | 200 | 1,0 | 3,0 | 1,3 | 2,0 | 1,83 |

Beispiel S:

| Verbindung | Dosierung g AS/ha | Herbizide Wirkung (Skala 1 -9) | | | | |
|---|---|---|---|---|---|---|
| | | ANTAR | CENAR | LAPCO | GALAP | ø |
| Stand. | 1000 | 1,1 | 5,0 | 1,3 | 2,0 | 2,35 |
| Verb. 99 | 500 | 1,0 | 1,9 | 1,0 | 3,7 | 1,9 |

Beispiel T:

| Verbindung | Dosierung g/ AS/ha | Herbizide Wirkung (Skala 1 - 9) | | | | |
|---|---|---|---|---|---|---|
| | | ANTAR | CENAR | LAPCO | GALAP | ø |
| Standard | 500 | 1,3 | 2,7 | 2,2 | 1,4 | 1,9 |
| Verb. 36 | 500 | 1,0 | 2,8 | 2,4 | 1,0 | 1,8 |
| Verb. 22 | 500 | 1,0 | 3,2 | 1,0 | 2,3 | 1,88 |
| Verb. 5 | 500 | 1,0 | 3,3 | 1,0 | 2,3 | 1,9 |

Beispiel U:

| Verbindung | Dosierung g AS/ha | Herbizide Wirkung (Skala 1 -9) | | | | |
|---|---|---|---|---|---|---|
| | | ANTAR | CENAR | LAPCO | GALAP | ø |
| Stand. | 750 | 3,9 | 5,3 | 2,9 | 2,9 | 3,75 |
| Verb. 54 | 750 | 2,7 | 2,9 | 2,7 | 3,5 | 2,95 |
| Verb. 53 | 750 | 2,6 | 4,7 | 2,8 | 4,3 | 3,6 |

**Patentansprüche**

**1.** Verfahren zur Bekämpfung von Unkräutern, dadurch gekennzeichnet, daß man mindestens eine Verbindung der Formel

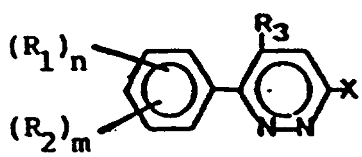 I

in der $R_1$ und $R_2$ unabhängig voneinander jeweils die Reste Hydroxy, Halogen, Cyano, Nitro, Amino, das gegebenenfalls durch Alkyl mit 1 - 4 C-Atomen substituiert sein kann, geradkettiges oder verzweigtes Alkyl mit 1 - 10 C-Atomen, das gegebenenfalls durch Hydroxy, Halogen oder Cyano ein oder mehrfach substituiert sein kann, geradkettiges oder verzweigtes Alkoxy mit 1 - 10 C-Atomen, Cycloalkyl mit 5 - 7 C-Atomen, Cycloalkoxy mit 5 - 7 C-Atomen, Phenyl, Phenoxy, Alkylthio oder Alkylsulfonyl mit 1 - 4 C-Atomen bedeuten,
n und m unabhängig voneinander jeweils die Zahlen 0, 1, 2 oder 3 bedeuten, wobei n + m die Zahlen 1 bis 5 bedeuten,
$R_3$ die Reste Hydroxy, Halogen, $-OC(O)ZR_4$ oder $-OC(O)NR_4R_5$ bedeutet, wobei
$R_4$ einen geradkettigen oder verzweigten gesättigten oder ungesättigten Alkylrest mit 1 - 15 C-Atomen

der gegebenenfalls durch einen Phenylrest substituiert sein kann oder einen Phenylrest oder einen Cycloalkylrest mit 5 - 7 C-Atomen
$R_5$ einen geradkettigen oder verzweigten gesättigten oder ungesättigten Alkylrest mit 1 - 15 C-Atomen der gegebenenfalls durch einen Phenylrest substituiert sein kann oder einen Phenylrest oder einen Cycloalkylrest mit 5 - 7 C-Atomen
Z eine Einfachbindung, Sauerstoff oder Schwefel und X Halogen bedeutet oder deren pflanzenphysiologisch verträgliche Salze auf Unkräuter oder deren Lebensraum einwirken läßt.

**2.** Verfahren nach Anspruch 1, dadurch gekennzeichnet, daß man mindestens eine Verbindung der Formel I, nach Anspruch 1, in der $R_1$ und $R_2$ unabhängig voneinander die Reste Halogen, geradkettiges oder verzweigtes Alkyl mit 1 - 4 C-Atomen, das gegebenenfalls ein- oder mehrfach durch Halogen substituiert sein kann, oder geradkettiges oder verzweigtes Alkoxy mit 1 - 4 C-Atomen bedeuten und n + m die Zahlen 1 oder 2 ist, oder einen Cyclohexylrest bedeutet, wobei n + m die Zahl 1 ist, $R_3$ den Rest -OC(O)Z$R_4$ bedeutet, wobei $R_4$ und Z die in Anspruch 1 angegebene Bedeutung haben, und X Chlor und Brom bedeutet oder deren pflanzenphysiologisch verträgliche Säureadditionssalze auf Unkräuter oder deren Lebensraum einwirken läßt.

**3.** Verfahren nach Anspruch 1, dadurch gekennzeichnet, daß man mindestens eine Verbindung der Formel I in der $R_1$ und $R_2$ unabhängig voneinander Chlor oder Fluor und n + m die Zahlen 1 oder 2 oder Trifluormethyl, und n + m die Zahl 1 bedeuten, $R_3$ den Rest -OC(O)Z$R_4$ wobei $R_4$ und Z die in Anspruch 1 angegebene Bedeutung haben, und X Chlor oder Brom bedeutet auf Unkräuter oder deren Lebensraum einwirken läßt.

**4.** Verwendung einer oder mehrerer Verbindungen der Formel I nach Anspruch 1 als Wirkstoff in einem herbiziden Mittel.

## Claims

**1.** Method of controlling weeds, characterized in that at least one compound of the formula

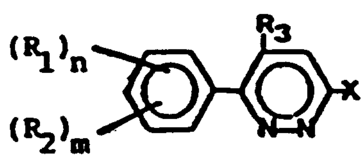 I

in which $R_1$ and $R_2$ independently of one another in each case represent the radicals hydroxyl, halogen, cyano, nitro, amino which can optionally be substituted by alkyl having 1 - 4 C atoms, or represent straight-chain or branched alkyl having 1 - 10 C atoms which can optionally be monosubstituted or polysubstituted by hydroxyl, halogen or cyano, or represent straight-chain or branched alkoxy having 1 - 10 C atoms, cycloalkyl having 5 - 7 C atoms, cycloalkoxy having 5 - 7 C atoms, phenyl, phenoxy, alkylthio or alkylsulphonyl having 1 - 4 C atoms, n and m independently of one another in each case represent the numbers 0, 1, 2 or 3, n + m representing the numbers 1 to 5,
$R_3$ represents the radicals hydroxyl, halogen, -OC(O)Z$R_4$ or -OC(O)N$R_4R_5$ where
$R_4$ represents a straight-chain or branched, saturated or unsaturated alkyl radical which has 1 - 15 C atoms and which can optionally be substituted by a phenyl radical, or represents a phenyl radical or a cycloalkyl radical having 5 - 7 C atoms,
$R_5$ represents a straight-chain or branched, saturated or unsaturated alkyl radical which has 1 - 15 C atoms and which can optionally be substituted by a phenyl radical, or represents a phenyl radical or a cycloalkyl radical having 5 - 7 C atoms,
Z represents a single bond, oxygen or sulphur and X represents halogen, or thereof a phytophysiologically acceptable salt is allowed to act on weeds or their environment.

**2.** Method according to Claim 1, characterized in that at least one compound of the formula I according to Claim 1, in which $R_1$ and $R_2$ independently of one another represent the radicals halogen, straight-chain or branched alkyl which has 1 - 4 C atoms and which can optionally be monosubstituted or polysubstituted by halogen, or represents straight-chain or branched alkoxy having 1 - 4 C atoms, and

n + m is the number 1 or 2, or represents a cyclohexyl radical where n + m is the number 1, $R_3$ represents the radical -OC(O)Z$R_4$ where $R_4$ and Z have the meaning given in Claim 1, and X represents chlorine and bromine, or a phytophysiologically acceptable acid addition salt thereof is allowed to act on weeds or their environment.

3. Method according to Claim 1, characterized in that at least one compound of the formula I, in which $R_1$ and $R_2$ independently of one another represent chlorine or fluorine and n + m represent the numbers 1 or 2, or represent trifluoromethyl, and n + m represent the number 1, $R_3$ represents the radical - OC-(O)Z$R_4$ where $R_4$ and Z have the meaning given in Claim 1, and X represents chlorine or bromine is allowed to act on weeds or their environment.

4. Use of one or more compounds of the formula I according to Claim 1 as active substance in a herbicidal composition.

## Revendications

1. Procédé pour lutter contre les mauvaises herbes, caractérisé en ce qu'on laisse agir, sur des mauvaises herbes ou leur biotope, au moins un composé de formule :

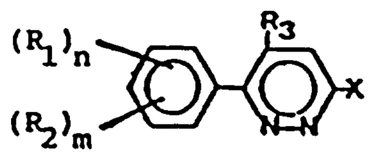

I

où $R_1$ et $R_2$ signifient chacun, indépendamment l'un de l'autre, un reste hydroxy, halogéno, cyano, nitro ou amino, qui peut être éventuellement substitué par un groupe alkyle ayant de 1 à 4 atomes de carbone, un groupe alkyle en $C_1$-$C_{10}$ à chaîne droite ou ramifiée, qui peut être éventuellement mono- ou polysubstitué par un groupe hydroxy, halogéno ou cyano, un groupe alcoxy en $C_1$-$C_{10}$ à chaîne droite ou ramifiée, un groupe cycloalkyle en $C_5$-$C_7$, cycloalcoxy en $C_5$-$C_7$, phényle, phénoxy, alkylthio ou alkylsulfonyle en $C_1$-$C_4$,
n et m signifient chacun, indépendamment l'un de l'autre, les nombres 0, 1, 2 ou 3, n + m valant de 1 à 5,
$R_3$ signifie les restes hydroxy, halogène, -OC(O)Z$R_4$ ou-OC(O)N$R_4$$R_5$, où
$R_4$ signifie un reste alkyle ayant de 1 à 15 atomes de carbone, saturé ou non saturé, à chaîne droite ou ramifiée, qui peut être éventuellement substitué par un reste phényle, ou un reste phényle ou un reste cycloalkyle ayant de 5 à 7 atomes de carbone,
$R_5$ signifie un reste alkyle ayant de 1 à 15 atomes de carbone, saturé ou non saturé, à chaîne droite ou ramifiée, qui peut être éventuellement substitué par un reste phényle, ou un reste phényle ou un reste cycloalkyle ayant de 5 à 7 atomes de carbone,
Z signifie une liaison simple, un atome d'oxygène ou de soufre,
et X signifie un halogène,
ou ses sels physiologiquement acceptables pour les plantes.

2. Procédé selon la revendication 1, caractérisé en ce qu'on laisse agir, sur des mauvaises herbes ou leur biotope, au moins un composé de formule I, selon la revendication 1, où $R_1$ et $R_2$ signifient, indépendamment l'un de l'autre, un reste halogéno ou un reste alkyle ayant de 1 à 4 atomes de carbone, à chaîne droite ou ramifiée, qui peut éventuellement être mono- ou polysubstitué par un halogène, ou un reste alcoxy ayant de 1 à 4 atomes de carbone, à chaîne droite ou ramifiée, et n + m vaut 1 ou 2, ou un reste cyclohexyle, auquel cas n + m vaut 1, $R_3$ signifie le reste -OC(O)Z$R_4$, où $R_4$ et Z ont la signification indiquée dans la revendication 1, et X signifie le chlore ou le brome, ou ses sels d'addition d'acide physiologiquement acceptables pour les plantes.

3. Procédé selon la revendication 1, caractérisé en ce qu'on laisse agir, sur des mauvaises herbes ou leur biotope, au moins un composé de formule I, où $R_1$ et $R_2$ signifient, indépendamment l'un de l'autre, le chlore ou le fluor et n + m vaut 1 ou 2, ou un reste trifluorométhyle, et n + m vaut 1, $R_3$ signifie le reste -OC(O)Z$R_4$, où $R_4$ et Z ont la signification indiquée dans la revendication 1, et X signifie le chlore

ou le brome.

**4.** Utilisation d'un ou plusieurs composés de formule I selon la revendication 1, comme substance active dans un agent herbicide.